(19) Europäisches Patentamt
European Patent Office
Office européen des brevets

(11) Publication number: **0 240 257 A2**

(12) **EUROPEAN PATENT APPLICATION**

(21) Application number: **87302644.7**

(22) Date of filing: **26.03.87**

(51) Int. Cl.⁴: **A61K 31/19**, A61K 31/275, A61K 31/215, A61K 31/335, A61K 31/12, A61K 31/16, A61K 31/34, A61K 31/40, A61K 31/405, A61K 31/365, A61K 31/52

(30) Priority: **27.03.86 GB 8607749**

(43) Date of publication of application: **07.10.87 Bulletin 87/41**

(84) Designated Contracting States: **AT BE CH DE ES FR GB GR IT LI LU NL SE**

(71) Applicant: **University of Lancaster**
**Bailrigg**
**Lancaster Lancashire LA1 4YW(GB)**

(72) Inventor: **Hetherington, Alistair MacCulloch**
**113 Scotforth Road**
**Lancaster Lancashire(GB)**
Inventor: **Huddart, Henry**
**13 Lunesdale Drive Forton**
**Preston Lancashire, PR3 0BH(GB)**
Inventor: **Mansfield, Terence Arthur**
**25 Wallace Lane Forton**
**Preston Lancashire, PR3 0BA(GB)**
Inventor: **Langton, Philip David**
**731 Plumas Street Apt. E**
**Reno Nevada 89509(US)**

(74) Representative: **Senior, Janet et al**
**Abel & Imray Northumberland House 303-306**
**High Holborn**
**London WC1V 7LH(GB)**

(54) **Compounds and pharmaceutical preparations having calcium - regulatory activity.**

(57) Pharmaceutical compositions useful in regulating calcium transport across cellular membranes in man and animals are disclosed. The compositions contain plant growth regulators such as abscisic acid and its derivatives. In particular, the compositions contain a compound of the formula:

$$\begin{array}{ccccccccc} & & & & R_5 & & R_6 & & \\ & & & & | & & | & & \\ R_1 & - & Xn & - & C & - & C & - & R_2 \\ & & & & | & & | & & \\ & & & & R_3 & & R_4 & & \end{array}$$

or

$R_1$ -Y - $R_2$

wherein $R_1$ is an alkyl of up to seven carbons or a saturated or unsaturated six membered carbon ring,

EP 0 240 257 A2

which may be substituted; $R_2$ is a cyano group, a thio or hydroxy lower alkyl group or a group containing a carbonyl, most commonly a carboxyl group; $R_3$ is hydrogen or lower alkyl; $R_4$ is hydrogen, lower alkyl or halide; $R_5$ is hydrogen or hydroxy; $R_6$ is hydrogen, carboxy or carboxy lower alkyl, or $R_5$ and $R_6$ form a bond between the carbons to which they are attached or with the carbons to which they are attached form a carbon ring; or $R_2$ and $R_6$ together with the carbon to which they are bonded form a heterocyclic ring; n is 0 or 1, with the proviso that when n is 0, $R_2$ is an acyl group; X is $-CH_2-$, $-CH=CH-$, $-CH_2-CH_2-$, $-CH_2-NH-$, $-CH_2-O-$, $-CH_2-S-$or $-NH-CO-$; and Y is a carbocyclic group bonded to $R_1$ and $R_2$ through a single carbon. The compositions are formulated for oral, parenteral or other form of administration in dosages necessary to effect the desired change in calcium flux. Daily doses of 0.01 to 200 mg/kg in one or more doses are contemplated. Dosage units may be formulated with any pharmaceutically acceptable carrier which is compatible with the active ingredient.

# Compounds and pharmaceutical preparations having calcium-regulatory activity

## Field of the Invention

The present invention relates to compounds and pharmaceutical preparations having calcium-regulatory activity. In particular, the invention is directed to use of plant growth regulators, specifically phytohormones and synthetic phytohormones such as abscisic acid ("ABA") and derivatives and metabolites thereof in human or animal disorders which respond to a change in calcium flux, to pharmaceutical compositions containing such compounds, and to the manufacture of such compositions.

## State of the Art

In the 1960s a number of substances isolated from plants were shown to have growth-regulating effects in the plant. Abscisic acid is one such compound which has been found to influence a number of processes in plants, including promotion of stomatal closure.

Our work with ABA has been concerned inter alia with probing the underlying mechanism for its action at the cellular level in higher plants, and recent work by De Silva, Co, Hetherington & Mansfield, reported in New Phytol., 101, 555-563, 1985, suggests that ABA may increase the permeability of the cell membrane to calcium ions, which could then act as secondary messengers interacting with the protein, calmodulin.

We have found that, surprisingly, the compound has an effect on calcium-regulation in animal cells, which is of potential pharmaceutical use. For example, in all muscles the maintenance of contractile activity depends upon the mobilization of intracellular calcium. Evidence from a number of studies shows that in smooth muscle and cardiac muscle of the vertebrates the initiation of muscular contraction commences with an inward calcium flux which subsequently releases stored cell calcium (see Cheng Jap. J. Pharmac. 26, 73-78, 1976; Fabiato & Fabiato, Ann. Rev. Physiol. 41, 473-484, 1979; and Huddart et al., J. Physiol. 349, 183-194, 1984).

At rest, the cell has a calcium concentration of $10^{-7}$M and the plasma has a calcium concentration of 2.5 mM. The consequent inward passive flux of calcium is balanced by the action of an ATPase in the membrane, which pumps calcium out. To counteract the resulting tendency to depolarize the cell, there is an inward flux of sodium and a matching transfer of sodium outward against potassium inwards.

For contraction of a muscle cell a sudden massive increase in the calcium level inside the cell is required. When muscular activity is initiated, a transmitter substance, for example acetylcholine, is released in response to an impulse arriving at a nerve ending and combines with a receptor in the plasma membrane. This receptor operates a channel (a 'receptor operated channel') in the plasma membrane, allowing accelerated calcium (and in some instances sodium) entry into the cell. The subsequent depolarization inactivates this fast receptor-operated channel but in turn activates slow voltage-dependent channels. These supply calcium to muscle fibres for sustained activity.

Various disorders, including angina and spasm in the major arteries, are thought to be due to excessive calcium entry into the cells and much research has been carried out by the pharmaceutical industry to discover calcium antagonists, that is, agents that block the inward movement of calcium into muscle cells, especially through slow voltage-dependent channels. Verapamil and nifedipine are two antagonists that are currently marketed. These interact with the slow calcium channels of the plasma membrane, inhibiting calcium entry and relieving the symptoms.

In some situations, however, for example heart failure, there is thought to be a need to increase calcium entry into the cell. However, considerable work in this field by the pharmaceutical industry has had only limited success, although the compound methyl 1,4-dihydro-2,6-dimethyl-5-nitro-4-[2-(trifluoromethyl)-phenyl]-3-pyridinecarboxylate, also known as BAY K 8644, mentioned in Example 7 of Bayer's European Patent 2208 B and U.S. Patent No. 4,248,873 and in Example 27 of GB specification 2,105,989 A is currently being evaluated for possible clinical use as a calcium agonist.

We have now found that another compound, ABA, also appears to act as a calcium agonist in animals. This plant growth regulator has been known for some time, but its presence has only recently been detected in animals; very recently published results suggest that the compound is endogenous in animals. Previous research has not found any pharmaceutical use for the compound related to calcium regulatory activity in animals.

Thus, in "Abscisic Acid", edited by F.T. Addicott, Praeger, 1983, Chapter 15, Visscher discusses his work on ABA and reviews the work of other animal physiologists on the compound and concludes that, at certain specific concentrations, ABA may play important, although incompletely defined, roles in animal metabolism through which it can inhibit the growth and reproduction of phytophagous animals that ingest it. For example, Visscher refers to observations in which ABA was added to healthy grasses grown in a greenhouse and fed to reproducing adults of A. elliotti and X. corallipes which demonstrated that ABA can produce immediate, dose related, growth-retarding effects on the developing embryonic progeny that could account for significant decreases in numbers of viable eggs produced by these females. Visscher also describes an experiment demonstrating a tendency towards a reduced number of pregnancies in female mice in groups treated with certain concentrations of ABA. U.S. Patent No. 4,209,530 describes and claims the use of ABA and its derivatives as insect control agents. Livingston's U.S. Patent No. 3,958,025 describes and claims the use of ABA and its analogues as a vitamin and also states that it has a marked effect on the inhibition of certain leukemia tumors in mice. However, we know of no work suggesting that ABA or its derivatives have calcium regulatory action in animals.

The present invention is directed to the use of (i) ABA, a pharmaceutically suitable derivative or appropriate human or animal metabolite thereof or (ii) a pharmaceutically suitable derivative of ABA capable of breakdown in the human or animal body to form ABA or an appropriate human or animal metabolite thereof or (iii) a pharmaceutically suitable cytokinin, gibberellin or auxin or a deriva tive or metabolite thereof, in a medicament having an effect on calcium movement across a cellular membrane, especially across the smooth or cardiac muscle membrane. Thus, the present invention provides the use of ABA or a pharmaceutically suitable derivative of ABA or other pharmaceutically suitable plant hormones or synthetic plant hormones or derivatives thereof (i) for the therapeutic or prophylactic treatment of, or for relief of symptoms of, disorders of the human or animal body due to abnormalities in calcium transport across a cellular membrane, especially across the smooth or cardiac muscle membrane, (ii) in surgery where an increase or decrease of calcium flux across membranes is required, and (iii) for tissue preservation or modulation of platelet aggregation.

Although abscisic acid and many of its derivatives are known compounds, prior to this invention no one had recognized the utility of these compounds as pharmaceuticals for use in controlling calcium flux in man and animals. There had been no suggestion for formulating these compounds in a form suitable for treating patients in whom control of calcium transport across cellular membranes is required for purposes of surgery or therapy. Nor had there been any suggestion of the methods of administering these compounds for such purposes.

## Summary of the Invention

This invention relates to the use of compositions containing certain pharmaceutically acceptable compounds, many of which have plant growth regulating activity, to treat human or animal disorders susceptible to treatment by a calcium regulator and to the manufacture of such compositions. Among the compounds which are contemplated for such use are those having the following formulae:

$$\text{I} \qquad R_1 - X_n - \underset{\displaystyle R_3}{\overset{\displaystyle R_5}{\underset{|}{\overset{|}{C}}}} - \underset{\displaystyle R_4}{\overset{\displaystyle R_6}{\underset{|}{\overset{|}{C}}}} - R_2$$

or

II $R_1$ -Y - $R_2$

wherein:

$R_1$ is selected from the group consisting of (i) saturated and unsaturated six-membered carbocyclic groups, which may be substituted or unsubstituted, and (ii) lower alkyl groups of up to seven carbons;

n is an integer selected from 0 and 1;

X is selected from the group consisting of $-CH_2-$, $-CH=CH-$, $-CH_2-NH-$, $-CH_2-O-$, $-CH_2-S-$, $-CH_2-CH_2-$, $-NH-CO-$;

$R_2$ is selected from the group consisting of (i) carboxyl and lower alkyl or phenyl esters and salts thereof, (ii) cyano, (iii) carbamoyl groups of the formula $CON(R_7\ R_8)$ wherein $R_7$ and $R_8$ are independently lower alkyl

or hydrogen, or with the nitrogen atom to which they are attached $R_7$ and $R_8$ form a saturated or unsaturated heterocyclic ring which may contain if desired one or more additional hetero atoms, (iv) hydroxy-or thio-lower alkyl, and (v) when n is 0, an acyl group of the formula $COR_9$ wherein $R_9$ is lower alkyl;
$R_3$ is hydrogen, lower alkyl or trifluoromethyl;
$R_4$ is selected from the group consisting of hydrogen, lower alkyl and halide;
$R_5$ is hydrogen or hydroxy;
$R_6$ is hydrogen, carboxy, or carboxy lower alkyl;
or $R_5$ and $R_6$ form a bond between the carbons to which they are bonded (that is, the carbons to which $R_5$ and $R_6$ are bonded have a double bond between them) or together with the carbons to which they are bonded form a saturated or unsaturated carbocyclic group, preferably of three or six members such as phenyl or cyclopropyl,
or $R_2$ and $R_6$ together with the carbon to which they are bonded form a heterocyclic group of up to six members, having therein at least one carbonyl group and preferably nitrogen as the heteroatom;
Y is a three to six membered carbocyclic group bonded to $R_1$ and $R_2$ through a single carbon of the ring.

The optical and geometric isomers of the compounds included within the above formulae are intended for use in the invention. Also contemplated for use in the present invention are compounds capable of breakdown in the human or animal body to form one of the compounds of the above formula and metabolites of the compounds of the above formula.

These compounds have calcium agonist or antagonist activities. Such activities make them useful in the treatment of various disorders including, among others, angina, hypertension, arrythmia, asthma, congestive heart failure and bone or bladder disease. The compounds having antagonist activity are also useful in tissue preservation and modulation of platelet aggregation.

## Brief Description of the Drawings

FIGURES 1 to 5 show results of tests of compounds of the invention, which tests are described in detail in the Examples.

## Detailed Description of the Invention

The present invention is directed to the use of compounds of the formulae set forth above to alter or regulate calcium flux across cell membranes in man or animals. More broadly the invention is concerned with the use of the above compounds, as well as any other plant growth regulators which affect calcium transport across cell membranes in plants, to effect some change in calcium flux across cell membranes in man and animals. The invention is also directed to pharmaceutical compositions which contain the compounds, to the manufacture of such compositions, to formulation of pharmaceutical compositions which have utility as calcium agonists or antagonists, and to the methods of treating man and animals with such compositions.

Particular compounds which have utility in the present invention include abscisic acid and its pharmaceutically acceptable derivatives, cytokinins, giberellins and auxins. Many of the compounds of the invention have been broadly defined by the formulae above. Examples of permissible substituents are more specifically defined below.

The definitions of $R_1$ and $R_2$ are the same for both formulae. $R_1$ is either an alkyl, most commonly a seven-membered alkyl, or a six-membered carbocyclic group. The ring may be fully saturated or have one, two, or three double bonds. In the case of three double bonds, $R_1$ is, of course, phenyl. The ring may be substituted by one or more of the same or different substituents or may be unsubstituted. Examples of substituents on $R_1$ include hydroxy, thio, oxo, lower alkyl, lower alkoxy, lower alkyl thio, trifluoromethyl, halide, carboxyl esters, epoxy, epidioxide, semicarbazono, lower alkylene and alkenylene bridge groups, and the bivalent group -C(O)-O-C(O)-. The substituents may be at any position on $R_1$.

Commonly, the substituents on $R_1$ are positioned in a manner similar to that of the substituents on the cyclohexenyl ring of abscisic acid. For ease of reference, the locations of $R_1$ substituents referred to herein is by reference to their location relative to substituents on the cyclohexenyl ring of abscisic acid rather than according to the hierarchy governing numbering for purposes of nomenclature. Thus the carbon to which the dimethyl groups are attached in abscisic acid is referred to as the 6-position in this general discussion of placement of substituents on $R_1$.

The following discussion of substitutents on the $R_1$ group is intended to be by way of example. Typically in the compounds used in the invention when $R_1$ is a carbocyclic group the 6-position is unsubstituted or has two methyl substituents. At the 4-position, an oxo, hydroxy, lower alkoxy, such as methoxy, or a semicarbazono may be present. An oxo substituent may also be present at the two or three position. At the 2-position lower alkyl, preferably methyl, or hydroxy, may be present. Halide substituents may be present at any one or more of the 1, 2 or 4 positions. As halides, bromo and/or chloro substituents may be present, although chloro substitution is more common. Epoxy substituents most typically are positioned between the one and two positions and -C(O)-O-C(O)-substituents between the two and three positions. Epidioxide, alkenylene and alkylene bridging groups generally are positioned between the one and four positions, and include for example $-CH=C(CH_3)-$with the $C(CH_3)$ group bonded to the one-position. Other substituents at the one position commonly include hydroxy, lower alkyl, and lower alkyl carboxyl ester groups. Unsaturation is most commonly either complete or a single double bond between the two and three positions. In the case where $R_1$ is phenyl, substituents are preferably selected from hydroxy, lower alkoxy (e.g. methoxy) and halogen (e.g. chloro and bromo). Hydroxy and lower alkoxy substituents may be, for example, in the 4-position and halogen, e.g. chloro, in the 2, 3 and/or 4-position.

$R_2$ contains at least one carbonyl function, except in the case of the cyano group or the hydroxy or thio lower alkyl groups. Generally $R_2$ is a carboxyl group or an ester thereof. Esters are generally either lower alkyl substituted or unsubstituted phenyl, or phenyl lower alkyl esters. When n is 0, $R_2$ must be an acyl group such as $C(CH_3)O$. When $R_2$ is the group $CON(R_7R_8)$ with $R_7$ and $R_8$ forming a heterocyclic ring, the ring preferably has five or six members. Examples of suitable heterocyclic groups include imidazole, thiazole, oxazole, and pyrrolidine.

Commonly $R_5$ and $R_6$ together are a covalent bond between the carbons to which they are attached. When that is not the case, they are generally hydrogen although the substituents set forth above are permissible. $R_4$ is most commonly hydrogen. $R_3$ is most typically methyl. When $R_4$ is halide it is generally chloride.

Y may be a ring of three to six carbons. Most commonly Y is cyclopropyl.

X is most typically $-CH=CH-$. It is to be understood that when X contains a hetero atom that the group is oriented from left to right as written herein with the right hand atom oriented toward $R_2$ and the left-hand atom oriented toward $R_1$.

Throughout this discussion references to lower alkyl, lower alkoxy, lower alkylene, lower alkenylene and the like are to be understood to include groups having one to four carbons in a chain on which one or more additional methyl groups may be substituents, except in those instances where longer chains are expressly included. Both straight and branched chain groups are included within the terms alkyl, alkoxy, alkylene and alkenylene. Preferred alkyls, alkoxy, alkylene and alkenylene groups have one to three carbons. Methyl is intended to encompass the group $CD_3$ as well.

Many of the compounds emcompassed within the present invention include one or more of the functional groups found in abscisic acid. The naturally occurring form of abscisic acid is the compound (+)-(1'S,2Z,4E)-5-(1'-hydroxy-2',6',6'-trimethyl-4'oxocyclohex-2'-enyl)-3-methylpenta-2,4-dienoic acid:

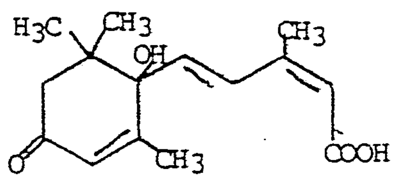

11

The term "abscisic acid" is used generally herein to include all stereoisomers of this compound. Any derivative of abscisic acid that has calcium-regulatory activity may be used. The term "derivative" is used herein to include, for example, analogues of the compound concerned (including structural isomers), and salts and functional derivatives, for example corresponding bases, of the compound and its analogues, and metabolites of the compound and of its other derivatives and compounds capable of breakdown in the human or animal body to form any of these. As applied to ABA, the term derivative includes the compounds included in the formulae set forth herein, whether or not such compounds have plant growth regulating activity.

An example of a derivative of ABA is (+)-vomifoliol. Other derivatives and analogues are described in Chapter 4 of "Abscisic Acid", edited by F.T. Addicott, which is incorporated by reference herein. Thus, suitable salts and esters of abscisic acid and optical isomers thereof may be utilized, for example, the methyl, phenyl, glucoside or glucose esters of abscisic acid. The 2-cis form of abscisic acid and its derivatives are of special interest. Further suitable abscisic acid derivatives are described and claimed in British patent specifications 1,073,882, 1,123,487, 1,123,488 and 1,164,564 (Shell), which are incorporated by reference herein.

Compounds contemplated for use in the present invention include those set forth in Tables I and II.

Table I

$$R_1 - X_n - \underset{R_3}{\underset{|}{C}} = \underset{R_4}{\underset{|}{C}} - R_2$$

| Compound | $R_1$ | n | X | $R_2$ | $R_3$ | $R_4$ | Preferred Isomers |
|---|---|---|---|---|---|---|---|
| ABA | 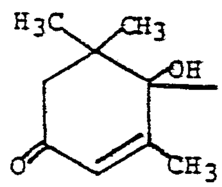 | 1 | CH=CH | COOH | $CH_3$ | H | 2Z, 4E |
| 1 | " | 1 | CH=CH | CN | $CH_3$ | H | 2Z, 4E |
| 2 | " | 1 | CH=CH | $COOCH_3$ | $CH_3$ | H | 2E, 4E |
| 3 | " | 1 | CH=CH | $COOC_6H_5$ | $CH_3$ | H | 2Z, 4E |
| 4 | " | 1 | CH=CH | CN | $CH_3$ | H | 2Z, 4E |
| 5 | " | 1 | CH=CH | COOn-$C_3H_7$ | $CH_3$ | H | 2Z, 4E |
| 6 | 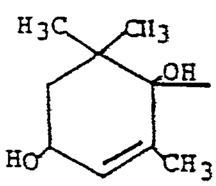 | 1 | CH=CH | COOH | $CH_3$ | H | 2Z, 4E 1'S, 4'R |
| 7 | " | 1 | CH=CH | $COOCH_3$ | $CH_3$ | H | 2Z, 4E 1'S |
| 8 | " | 1 | CH=CH | $COOCH_3$ | $CH_3$ | H | 2E, 4E 1'S, 4'R |
| 9 | " | 1 | CH=CH | COOH | $CH_3$ | H | 2E, 4E 4'R |

| Compound | $R_1$ | n | X | $R_2$ | $R_3$ | $R_4$ | Preferred Isomers |
|---|---|---|---|---|---|---|---|
| 10 | 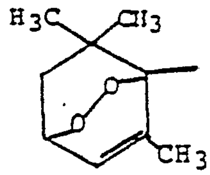 | 1 | CH=CH | COOH | $CH_3$ | H | 2Z, 4E<br>or<br>2E, 4E |
| 11 | " | 1 | CH=CH | $COOCH_3$ | $CH_3$ | H | 2E, 4E |
| 12 | 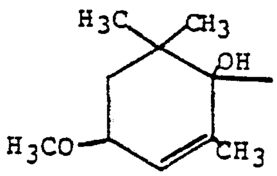 | 1 | CH=CH | $COOCH_3$ | $CH_3$ | H | 2Z, 4E<br>1'S, 4'S<br><br>2Z, 4E<br>1'S, 4'R |
| 13 | " | 1 | CH=CH | $CH_2OH$ | $CH_3$ | H | 2Z, 4E |
| 14 | 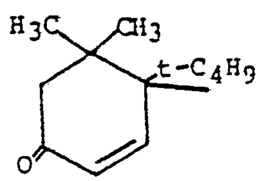 | 1 | CH=CH | COOn-<br>$C_3H_7$ | $CH_3$ | H | 2Z,4E |
| 15 | 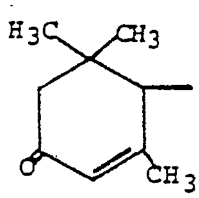 | 1 | CH=CH | $COOCH_3$ | $CH_3$ | H | 2E, 4E<br>2Z, 4E |
| 16 | 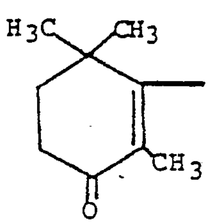 | 0 | - | $CH_3$<br>\|<br>CO | H | H | 3E |
| 17 | 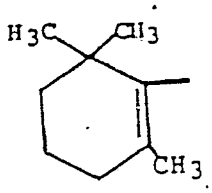 | 1 | CH=CH | $CONH_2$ | $CH_3$ | H | 2E, 4E |

| Compound | $R_1$ | n | X | $R_2$ | $R_3$ | $R_4$ | Preferred Isomers |
|---|---|---|---|---|---|---|---|
| 18 | " | 1 | CH=CH | COOH | $CH_3$ | H | 2E, 4E<br>2Z, 4E |
| 19 | 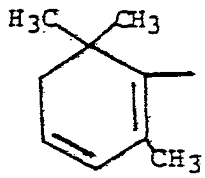 | 1 | CH=CH | COOH | $CH_3$ | H | 2Z, 4E |
| 20 | 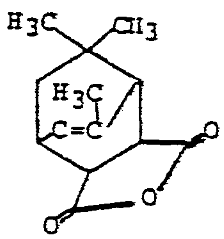 | 1 | CH=CH | $COOCH_3$ | $CH_3$ | H | 2E, 4E |
| 21 | 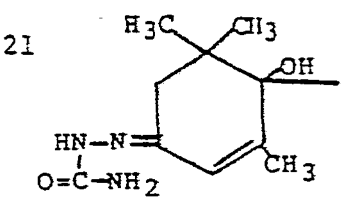 | 1 | CH=CH | COOH | $CH_3$ | H | 2Z, 4E |
| 22 | 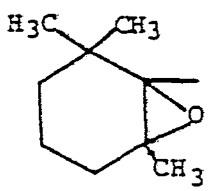 | 1 | CH=CH | COOH | $CH_3$ | H | 2Z, 4E<br>2E, 4E |
| 23 | " | 1 | CH=CH | $COOC_2H_5$ | $CH_3$ | H | 2E, 4E<br>2Z, 4E |
| 24 | " | 1 | CH=CH | $COOC_6H_5$ | $CH_3$ | H | 2Z, 4E |
| 25 | " | 1 | CH=CH | $COOCH_3$ | $CH_3$ | H | 2Z, 4E |
| 26 | " | 1 | CH=CH | $CONHCH_3$ | $CH_3$ | H | 2Z, 4E |
| 27 | " | 1 | CH=CH | COOH | $CF_3$ | H | 2Z, 4E |
| 28 | " | 1 | CH=CH | $COOC_2H_5$ | $CH_3$ | $CH_3$ | 2Z, 4E<br>2E, 4E |

| Compound | $R_1$ | n | X | $R_2$ | $R_3$ | $R_4$ | Preferred Isomers |
|---|---|---|---|---|---|---|---|
| 29 | " | 1 | CH=CH | $COOC_2H_5$ | $CH_3$ | Cl | 2Z, 4E<br>2E, 4E |
| 30 | " | 1 | CH=CH | COOH | $CH_3$ | $CH_3$ | 2E, 4E |
| 31 | " | 1 | CH=CH | $COOC_2H_5$ | H | H | 2E, 4E |
| 32 | " | 0 | - | CO<br>$CH_3$ | H | H | 3E |
| 33 | 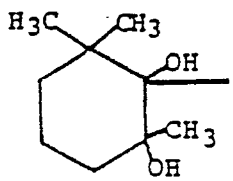 | 1 | CH=CH | COOH | $CH_3$ | H | 2E, 4E<br>2Z, 4E |
| 34 | 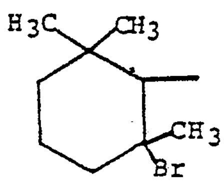 | 1 | CH=CH | COOH | $CH_3$ | H | 2Z, 4E |
| 35 | 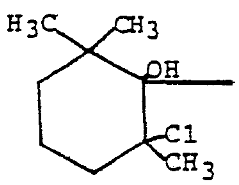 | 1 | CH=CH | COOH | $CH_3$ | H | 2Z, 4E<br>2E, 4E<br>1'S |
| 36 | 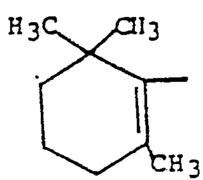 | 1 | CH=CH | $COOC_2H_5$ | H | H | 2E, 4E |
| 37 | 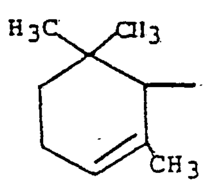 | 1 | CH=CH | COOH | $CH_3$ | H | 2Z, 4E |

| Compound | $R_1$ | n | X | $R_2$ | $R_3$ | $R_4$ | Preferred Isomers |
|---|---|---|---|---|---|---|---|
| 38 | 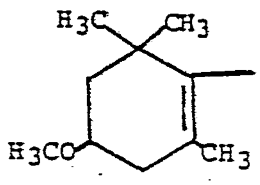 | 0 | - | CO<br>$CH_3$ | H | H | 3E |
| 39 | 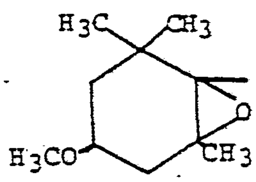 | 0 | - | CO<br>$CH_3$ | H | H | 3E |
| 40 | " | 1 | CH=CH | $COOC_2H_5$ | $CH_3$ | H | 4E |
| 41 | " | 1 | CH=CH | COOH | $CH_3$ | H | 2E, 4E |
| 42 | 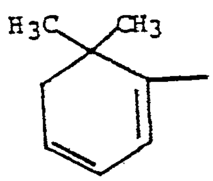 | 1 | CH=CH | COOH | $CH_3$ | H | 2Z, 4E |

Table II

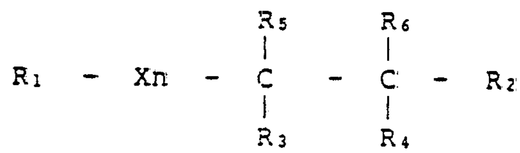

| Compound | R1 | n | X | R2 | R3 | R4 | R5, R6 |
|---|---|---|---|---|---|---|---|
| 43 | 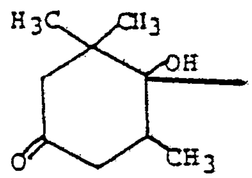 | 1 | $CH_2-CH_2$ | $COOCH_3$ | $CH_3$ | H | H, H |
| 44 | 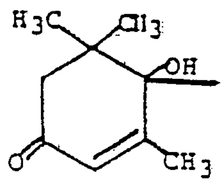 | 1 | $CH_2-CH_2$ | $COOCH_3$ | $CH_3$ | H | H, H |
| 45 | 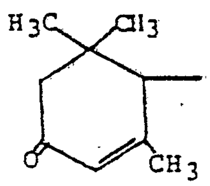 | 1 | CH=CH | $COOCH_3$ | $CH_3$ | H | OH, H |
| 46 | 4-methoxy-phenyl | 1 | $CH_2-CH_2$ | COOH | $CH_3$ | H | H, H |
| 47 | " | 1 | $CH_2-CH_2$ | COOH | H | H | H, H |
| 48 | 4-hydroxy-phenyl | 1 | $CH_2-CH_2$ | COOH | $CH_3$ | H | H, H |
| 49 | " | 1 | $CH_2-CH_2$ | COOH | H | H | H, H |
| 50 | 4-methoxy-phenyl | 1 | $CH_2-CH_2$ | COOH | H | H | H, COOH |
| 51 | 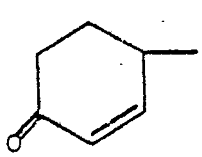 | 0 | - | CO-$CH_3$ | H | H | H, H |

| Compound | $R_1$ | n | X | $R_2$ | $R_3$ | $R_4$ | $R_5$, $R_6$ |
|---|---|---|---|---|---|---|---|
| 52 | 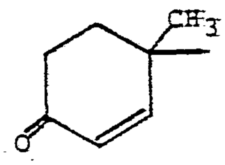 | 0 | - | CO<br>$CH_3$ | H | H | H, H |
| 53 | 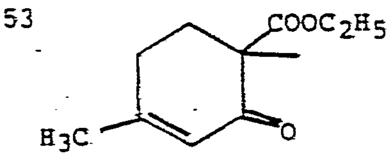 | 1 | $CH_2$ | $COOC_2H_5$ | H | H | H, H |
| 54 | 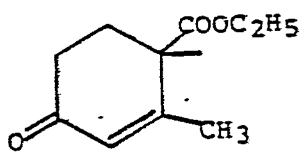 | 1 | $CH_2$ | $COOC_2H_5$ | H | H | H, H |
| 55 | 4-chloro-phenyl | 1 | NH-CO | COOH | H | H | $-CH_2-$* |
| 56 | " | 1 | $CH_2$-NH | COOH | [See $R_5$, $R_6$] phenyl** | | |
| 57 | normal-heptyl | 1 | CH=CH | COOH | H | H | H, $CH_2COOH$ |
| 58 | normal-heptyl | 1 | CH=CH | $CH_2$CONHCO * | H | H | H, [see $R_2$] |
| 59 | 2,4-dich-lorophenyl | 1 | NH-CO | COOH | H | H | $-CH_2-$* |
| 60 | 3,4-dich-lorophenyl | 1 | NH-CO | COOH | H | H | $-CH_2-$* |

* Represents a bridging group forming a ring with the carbon(s) to which the group is attached.

** Phenyl is formed by $R_3$, $R_4$, $R_5$ and $R_6$ and carbons to which they are attached.

In Table I reference to Preferred Isomers signifies isomers which have been made and does not reflect preferred activity relative to other isomers.

The invention also includes, by way of example of compounds containing Y, 1-(2,4-dichlorophenyl)-cyclopropane carboxylic acid. Other examples of compounds within the invention include gibberellic acid ($GA_3$), indole-3-acetic acid and dihydrozeatin.

Synthetic methods for the preparation of abscisic acid and its analogs are described in British Patent Nos. 1,073,882, 1,123,487, 1,123,488, and 1,164,564; U.S. Patent Nos. 2,827,481, 3,576,880, 3,793,375, and 3,803,217; J. Org. Chem., 22, 224 and 1611 (1957); Helv. Chim. Acta, 45, 528 (1962); Nature, 206, 715 (1965); Agr. Biol. Chem., 29, 962 (1965), 30, 434 (1966), 32, 1357 (1969), and 34, 1821 (1970); Chem. Commun., 1967, 114 (1967); Can. J. Chem., 49, 2369 (1971); and J. Amer. Chem. Soc., 95, 239 (1973). Broadly, the preparation of the compounds may be carried out, for example, as follows.

Treatment of a suitably substituted beta-ionone with meta-chloroperbenzoic acid will form the corresponding substituted cyclohexane-1,2-epoxide. Two-carbon extension of the side chain can be accomplished by the Wittig reaction using reagents such as triethyl phosphonoacetate. Opening of the epoxide under selective acidic conditions will provide the diol, or the bromo-or chlorohydrin.

Two-carbon extension of the side chain of a suitably substituted beta-ionone can be accomplished by the Wittig reaction using reagents such as triethyl phosphonoacetate. Bromination with NBS followed by dehydrobromination will give the corresponding substituted cyclohexa-1,2,3,4-diene, which when irradiated with oxygen present will form the 2,3-cyclohexene-1,4-epidioxide.

A suitably substituted 2,3-cyclohexene-1, 4-epidioxide when treated with aqueous base will rearrange to form the corresponding substituted 1-hydroxy-4-keto-2, 3-cyclohexene compound. Selective reduction of the ketone will form the 1,4-dihydroxy-2,3-cyclohexene compound. A suitably substituted 1-hydroxy-4-keto-2,3-cyclohexene compound can be converted to the 1-dehydroxy compound in three steps by protective blocking of the ketone group, selective dehydroxylation, and unblocking of the ketone. The substituted 4-keto-2,3-cyclohexene compound thus prepared can then be selectively reduced to form the corresponding substituted 4-hydroxy-2,3-cyclohexene compound.

Some aromatic analogs of abscisic acid can be prepared in three steps from a suitably substituted benzaldehyde precursor. For example, condensation of 4-methoxybenzaldehyde with acetone followed by the Reformatsky reaction with ethyl bromoacetate and dehydration with paratoluenesulfonic acid will provide ethyl 5-(4-methoxyphenyl)-3-methyl-2,4-pentadienoate.

The aromatic analogs containing spirocyclopropane in the side chain can be prepared in multiple steps from a suitably substituted benzene precursor. For example, the four-step sequence of Friedel-Crafts reaction of 1,3-dichlorobenzene with ethyl oxalyl chloride, reaction with methyl Grignard, acidic dehydration, and reaction with diiodomethane and zinc-copper couple will provide ethyl 1,1-(2′,4′-dichlorophenyl)-cyclopropanecarboxylate. The ester can be saponified to the corresponding acid.

Aromatic analogs containing nitrogen and cyclopropane in the side chain can be prepared from a suitably substituted aniline precursor. For example, acylation of 4-chloroaniline with maleic anhydride followed by reaction with diiodomethane and zinc-copper couple will provide N-(4-chlorophenyl)-cyclopropanamide-2-carboxylic acid.

Aromatic analogs containing nitrogen and a benzene ring in the side chain can be prepared in multiple steps from a suitably substituted aniline precursor. For example, coupling of 4-chlorobenzaldehyde with ethyl 2-aminobenzoate followed by selective reduction of the imine formed will provide ethyl N-(4-chlorobenzyl)-2-aminobenzoate. The ester can be saponified to the corresponding acid.

Compounds with a ten-carbon side chain can be prepared from 2-(2-decenyl)succinic anhydride or a suitably substituted analog. For example, treatment of 2-(2-decenyl)succinic anhydride with a base such as sodium methoxide will form 2-(2-decenyl)succinic acid; heating 2-(2-decenyl)succinic anhydride with ammonium hydroxide will form 2-(2-decenyl)succinimide.

It is not possible to predict whether a given compound useful in the invention is a calcium agonist or a calcium antagonist. Comparatively small changes in structure bring about significant differences, even reversal of, the calcium transport effect of a compound. For example, the compound nifedipine acts as a calcium antagonist; it also antagonizes the effects of ABA and the compound BAY K 8644. both nifedipine and the BAY K 8644 compound are dihydropyridine derivatives and have, respectively, the general formulae III and IV shown below.

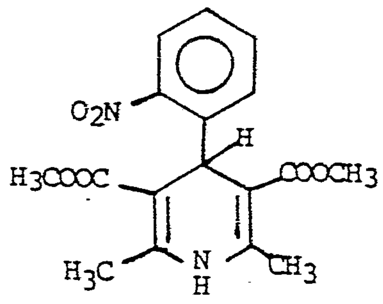

III

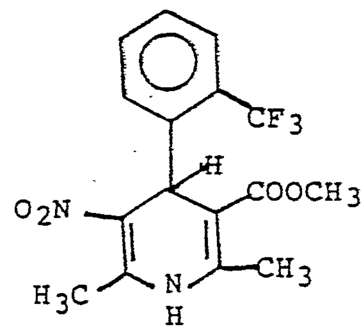

IV

Nonetheless, it is possible to establish whether or not particular derivatives have calcium agonist or antagonist activity by means of routine tests. Compounds which have an especially pronounced effect on calcium transport can also easily be ascertained by routine testing.

The mechanism of calcium entry to muscle fibres has been intensively studied by Sperelakis in Membrane Biochem. 5, 131-166, 1984; and by Hurwitz, 1986, Ann. Rev. Pharmacol. Toxicol., 26, 225-258. Calcium enters most visceral muscles from the plasma by two distinct channels - the so-called fast receptor operated channels and the slow voltage-dependent channels. These channels which gate calcium entry differ not only in their kinetics but in the cell membrane voltage differential ranges in which they operate. On activation it is initially the fast channels that operate, but, with the changing cell polarity thereby produced, these are auto-inactivated and the slow channels open. Contraction of the cell begins as the fast channel is inactivated and continues as the slow channel opens.

The slow calcium channels operate in a less negative voltage range than the fast channels and so the latter can be inactivated by $K^+$-depolarization. The slow channels can thus be separately studied by voltage-inactivation of the fast channels using salines with elevated $K^+$ levels.

In mammalian unitary smooth muscles, recent studies on the slow calcium channels have shown that these channels are readily blocked by a number of agents such as papaverine, verapamil, nifedipine and octylonium bromide (see Nayler & Poole-Wilson, Basic Res. Cardiol., 76, 1-15, 1981; Brading et al., J. Physiol., 334, 79-89, 1983; Huddart et al., J. Physiol., 349, 183-194, 1984, and Huddart & Butler, Gen. Pharmacol., 17, 695-703, 1986). Muscle fibre membrane slow calcium channels thus provide a convenient bioassay for agents which activate or inactivate calcium transport across membranes.

It is known that the rat vas deferens and bladder smooth muscles respond to field stimulation by electrodes with consistent repeatable contractions which are calcium-dependent via the operation of slow calcium channels, as is all contractile activity in these muscles (see Huddart et al. ibid., J. Exp. Biol., 107, 73-93, 1983, and Huddart & Butler, ibid.). We found that, at a concentration of $10^{-6}$M, ABA enhanced these field stimulation responses in both muscles by about 25%, (n = 16 in both cases). This augmentation (and the response itself) is inhibited by the slow channel blocker nifedipine.

In $K^+$-depolarized muscle where fast channels are voltage-inactivated, we found that ABA at $10^{-6}$M considerably augmented contracture tension and enhanced the tonic phase of the response. The latter is very strongly dependent upon slow calcium channel activity.

Another calcium-dependent activity is the contractile cycle of the vertebrate heart. In flounder heart ABA at $10^{-6}$M was found to augment contractile force, and the action was inhibited by nifedipine, while prolonged ABA exposure induced pronounced cardiac arrhythmia.

These results are remarkably similar to those obtained with BAY K 8644.

While these findings are very preliminary they do suggest that ABA may have general action on plasma membranes to augment calcium influx. The mechanism of ABA action can only be guessed at this juncture. Since ABA action is nifedipine-sensitive it is unlikely that ABA has an ionophore-like action - its activity seems quite unlike that of A 23187 (Calbiochem Catalog 1987), a synthetic $Ca^{2+}$ ionophore. A number of possibilities spring to mind:

(1) ABA may act to open previously closed channels;

(2) ABA may either increase channel open time or shorten channel closure time (as do some insect neuro-active peptides);

(3) ABA may actually augment channel kinetics to increase transport number;

(4) ABA may block the method by which the cell exports calcium; and

(5) ABA may induce calcium release from bound cellular stores.

Regardless of the mode of action, the present invention provides the manufacture and use of compositions containing ABA or a pharmaceutically suitable derivative thereof having calcium agonist effect to activate calcium transport across a cellular membrane in the human or animal body, and also the manufacture and use of compositions containing a pharmaceutically suitable derivative of ABA having calcium antagonist effect to deactivate calcium transport across a cellular membrane in the human or animal body. Use in the field of coronary and vascular diseases should especially be mentioned.

Possible uses of agonists are in situations where more contractile force is required in the muscle. General examples of conditions likely to be responsive to a calcium agonist include hypotension, congestive heart failure, gastrointestinal diseases, bladder diseases and bone diseases. More specific indications are, for example, in the case of loss of bladder tone or left ventricular failure, in the treatment of swelling in the mucous membrane, in the treatment of diseases involving a depression of blood sugar or incorrect salt and fluid balance, in the treatment of hypotensive circulatory conditions, or in the temporary restriction of peripheral blood flow during minor surgical procedures. The recent discovery that the hormonal-induced release of glucose from hepatocytes is calcium-stimulated (Woods, Cuthbertson & Cobbold, Nature, 319, 600-602, 1986) makes it clear that ABA and/or its derivatives may also have therapeutic use in liver dysfunction.

Possible uses of antagonists are as a peripheral vasodilator, e.g. to treat Raynaud's disease, to reduce hypertension, to treat angina, asthma, cardiovascular disease or cardiovascular or cerebrovascular system disturbances, to correct spasm in major blood vessels, in the bladder, or in the gastrointestinal tract, to correct cardiac arrythmia, and to treat migraine, atherosclerosis or osteoporosis. The calcium antagonists are also useful for tissue preservation, e.g. kidney, liver and heart, and for platelet aggregation.

Other uses of calcium agonists and antagonist are described, for example, in Calcium and Cell Physiology, edited by Dieter Marme, Springer-Verlag. The fact that plant growth regulators have wide-ranging action on animal tissues where calcium activation or deactivation is significant was previously unsuspected.

As indicated above, ABA operates not only as a calcium regulator in animals, but also appears to act in this way in plants in its action on the stomatal apparatus. It is known that the other plant growth regulators, the cytokinins, the gibberellins and auxins (indol-3-ylacetic acid (IAA) and related compounds), also affect the stomatal apparatus. Some of the compounds act antagonistically to others. For example, in contrast to ABA, IAA encourages stomatal opening. It is not yet known whether these other plant growth regulators work on plants by affecting the calcium channels.

The present invention is intended to include use of any pharmaceutically suitable natural or synthetic plant growth regulator, derivatives or metabolite thereof even those which are not derivatives of ABA, but which have calcium-regulatory activity in the human or animal body, for the therapeutic or prophylactic treatment of, or for relief of symptoms of, a disorder of the human or animal body susceptible to treatment by increase or decrease of the calcium flux across a cellular membrane, especially the smooth or cardiac muscle membrane, or for use in surgery where such increase or decrease is required. The compounds included within the invention may be natural or synthetic substances. Apart from ABA and its derivatives, the cytokinins should especially be mentioned.

The active ingredient may be administered one or more times, e.g. three times, per day. Dosages will typically be 0.01 to 200 mg/kg/day depending on the activity of the compound and the indication for which it is being given. For example, in humans dosages of 0.15 mg to 3g/day may be given. In the case of compositions containing supplementary active ingredients, the dosages are determined by reference to the usual dose and manner of administration of the said ingredients.

A medicament manufactured in accordance with the present invention may be in the form of a pharmaceutical preparation containing the active ingredient and a pharmaceutically suitable carrier or it may consist of the active ingredient per se in a form suitable for use in a pharmaceutical preparation, that is in substantially pure, and (in the case of the substance for injection) pyrogen-free, form and, in the case of a pharmaceutical preparation, manufac tured in accordance with the code of Good Manufacturing Practice of the pharmaceutical industry.

It is especially advantageous to formulate the preparations in dosage unit form for ease of administration and uniformity of dosage. "Dosage unit form" as used herein refers to physically discrete units suitably as unitary dosages for the subjects to be treated; each unit contains a predetermined quantity of active material calculated to produce the desired therapeutic effect. The specifications for the dosage unit forms of the invention are dictated by and directly dependent on (a) the unique characteristics of the active material and the particular therapeutic effect to be achieved, and (b) the limitations inherent in the art of compounding such an active material for the treatment of diseases in living subjects.

The formulation of the preparations of the invention may be carried out in a manner known per se by working up the active ingredient with the carrier substance, for example diluent, taste corrective (e.g. flavorant) or other additive(s) customarily used in galenical pharmacy, and conversion into the desired form of application.

As used herein, "pharmaceutically suitable (or acceptable) carrier" includes any and all suitable solvents, dispersion media, coatings, antibacterial and antifungal agents, isotonic and absorption delaying agents and other suitable auxiliaries. The use of such media and agents for pharmaceutically active substances is well known in the art. Except insofar as any conventional media or agent is incompatible with the active ingredient, its use in the therapeu tic preparations is contemplated. Supplementary active ingredients may also be incorporated into the preparations.

Pharmaceutically suitable carriers which may be used in the preparations of the present invention are, for example, starches, cellulose, dextran, chemically-modified cellulose, calcium carbonate, calcium phosphate, magnesium phosphate, silica, magnesium silicates, titanium silicates, talc and formulated casein for dry formulations; water, sugar syrups and human plamsa-isotonic salines for liquid formulations; and cocoa butter and polyethyleneglycol stearates for suppositories or topically applied creams. Of course, any material used in preparing any pharmaceutical preparation should be pharmaceutically pure and substantially non-toxic in the amounts employed.

The medicaments and preparations of the present invention may be in a form suitable for oral, parenteral, rectal, intravenous, percutaneous, permucous or sublingual administration, for example in the form of tablets, coated tablets, dragees, sachets, pills, capsules, soft gelatine capsules, syrups, drinkable suspensions or solutions packed in ampoules, multidose flasks or auto-injectable syringes or suppositories.

The content of the active ingredient in the pharmaceutical preparations will vary depending on the therapeutic use and the method of administration of the preparations. The content may also vary depending on the weight and the age of the patient to be treated and the severity of the disorder to be treated. The content may range, for example, from 0.05 mg to 10g per unit dosage form. More commonly individual dosage units may contain 0.5 mg to 1g of active, more particularly 1 mg to 250 mg. Individual dosage units may, however, contain as little as 60 mg or even as little as 10 mg. A common dosage may well be that used for other calcium antagonists, i.e., 5 to 20 mg. three times daily. For pharmaceutical preparations intended for parenteral or rectal administration, the amount of active ingredient will be at the lower end of the range.

When intended for oral administration, the active compounds may, for example, be formulated with an inert diluent or with an assimilable edible carrier, or they may be enclosed in hard or soft shell capsules, or they may be compressed into tablets with inert cellulose or dextran carriers, or they may be incorporated directly with the food of the diet. The active compounds may also be incorporated with excipients and used in the form of ingestible tablets, buccal tablets, troches, capsules, elixirs, suspensions, syrups or wafers. Generally, such preparations should contain at least 0.1% of active compound. The percentage in the preparations may, of course, be varied and may conveniently be from 2 to 60%, e.g. from 10 to 50%, of the weight of the unit. The amount of active compound in such therapeutically useful preparations is such that a therapeutically effective dosage will be obtained. Oral dosage unit form preparations may contain at least 1 mg to 1 g, more specifically 1 mg to 10 mg, of active compound.

The tablets, troches, pills, capsules and solid formulations for oral use may also contain, for example, one or more of the following: a binder, such as gum tragacanth, acacia, corn starch, dextran or gelatin; excipients, such as dicalcium phosphate; a disintegrating agent, such as corn starch, potato starch or alginic acid; a lubricant, such as magnesium stearate; a sweetening agent, such as sucrose, lactose, aspartame or saccharin; or a flavouring agent, such as peppermint, oil of wintergreen or cherry flavoring. When the dosage unit form is a capsule, it may contain for example, in addition to materials of the above type, a liquid carrier. Various other materials may be present as coatings or otherwise to modify the physical form of the dosage unit. For instance, tablets, pills or capsules may be coated with shellac, sugar or both. A syrup or elixir may contain the active compound, sucrose as a sweetening agent, methyl and propylparabens as preservatives, a dye and flavouring, such as cherry or orange flavour. In addition, the active compounds may be incorporated into sustained-release preparations and formulations. Thus, for example, for achieving a protracted action, the active ingredient may be used in a micro-encapsulated form.

In addition to oral administration, the active compounds may also be administered parenterally, intraperitoneally or subcutaneously. Solutions of the active compound may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations may contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. Particular attention may have to be paid to the photostability of the active ingredient and suitable opaque coatings or storage vessels may be employed. The carrier may be a solvent or dispersion medium containing, for example, water, ethanol, polyol (for example, glycerol, propylene glycol and liquid polyethylene glycol), suitable mixtures thereof and vegetable oils. The proper fluidity may be maintained, for example, by the use of a coating such, for example, as lecithin, by the maintenance of the required particle size in the case of dispersions and by the use of surfactants. The prevention of the action of microorganisms may be brought about by various antibacterial and antifungal agents, for example, parabens, chlorobutanol, phenol, sorbic acid or thimerosal. In many cases, it will be preferable to include isotonic agents, for example, sugars or sodium chloride. Prolonged absorption of the injectable compositions may be brought about by use of agents which delay absorption, for example aluminum monostearate and gelatin.

Sterile injectable solutions may be prepared by incorporating the active compound in the required amount in the appropriate solvent with various of the other ingredients enumerated above, as required, followed by filtered sterilization. Dispersions may be prepared by incorporating the sterilized active ingredient into a sterile vehicle which contains the basic dispersion medium and the required other ingredients from those enumerated above. In the case of sterile powders for the preparation of sterile injectable solutions, methods of preparation are, for example, vacuum drying and the freeze-drying technique which yield a powder of the active ingredient plus any additional desired ingredient from a previously sterile-filtered solution thereof.

Dosage unit forms suitable for intravenous administration may contain up to 500 mg, more particularly up to 10 mg, or even as little as 1 mg.

The medicaments may be in the form of a pack which includes instructions for their use.

The present invention provides a pharmaceutical preparation, which comprises a pharmaceutically suitable plant growth regulator or synthetic derivative thereof having calcium-regulatory activity, in admixture or conjunction with a pharmaceutically suitable carrier, the preparation being in dosage unit form and containing up to 250 mg, especially up to 60 mg, more particularly up to 10 mg, of active ingredient per dosage unit.

The present invention also provides a pharmaceutical preparation which comprises ABA or a derivative thereof having calcium regulatory activity, in admixture or conjunction with a pharmaceutically suitable carrier, the prepara tion being in dosage unit form and containing up to 250 mg, especially up to 60 mg, more particularly up to 10 mg, of active ingredient per dosage unit.

The present invention further provides a method of treatment of the human or animal body, which comprises administering to a human or non-human animal an effective, non-toxic, amount of a pharmaceutically suitable plant growth regulator or synthetic derivative thereof having calcium-regulatory activity in the human or animal body.

The present invention also provides a method of treatment of the human or animal body, which comprises administering an effective, non-toxic, amount of ABA or a pharmaceutically suitable derivative thereof having calcium-regulatory activity in the human or animal body to a human or non-human animal as calcium agonist or antagonist.

The following Examples illustrate the invention.

## Examples

Smooth muscle preparations were obtained from male Wistar strain albino rats. The animals were killed by a blow to the head and smooth muscle strips (about 2 mm x 10 mm) were dissected from the bladder. The vasa deferentia were divided into two portions and longitudinally bisected. Tests on cardiac muscle were carried out using isolated cannulated flounder heart. All mammalian preparations were maintained in Krebs solution containing

$NaCl$ : 120.7 mM; $NaH_2PO_4$ : 1.3 mM;

$KCl$ : 5.9 mM; $NaHCO_3$ : 15.5 mM and

$CaCl_2$ : 2.5mM; glucose : 11.5 mM.

$MgCl_2$ : 1.2mM

The Krebs solution was adjusted to pH 7.3 with HCl and was continuously aerated and maintained at 37°C.

The flounder heart was maintained at 10°C in flounder saline containing
NaCl : 116.7 mM, $CaCl_2$ : 1.72 mM,
$Na_2SO_4$ : 18.6 mM, TES : 2.5 mM and
KCl : 2.71 mM, glucose : 5.0 mM
$MgSO_4$ : 0.67 mM,
at pH 7.65.

To record tension the muscle preparations were mounted vertically in jacketed organ baths which were continuously aerated and whose contents could be rapidly changed. The preparations were attached to a set of Grass Instruments ST 0.3C force displacement transducers held in adjustable rackwork, and the racks were adjusted to put slight tension on the preparations. After mounting in the perfusion baths the preparations were allowed to equilibrate for 1 hour, the bath saline being changed every 15 min. The force displacement transducer outputs were connected to a four-channel Grass model 79 polygraph. The stimulation electrode terminals were attached to Grass SIU 5 isolation units connected to two Grass S48 stimulators driven in tandem. Stimulation train parameters varied in different preparations but a common regime consisted of a 200 msec train of pulses at 50 Hz, each pulse being 2-5 msec at 40-50 V. Trains were automatically repeated usually at 100 or 50 sec intervals. The techniques have been described in detail by Huddart and Butler (1986) in Gen. Pharmac., 17, 695-703 (1986).

Tests were carried out with synthetic abscisic acid (the racemic form of (1'*S*,2*Z*,4*E*)-5-(1'-hydroxy-2',6',6'-trimethyl-4'-oxocyclohex-2'-enyl)-3-methyl-penta-2,4-dienoic acid.

## Example 1

The smooth muscle preparations were threaded through two silver/silver chloride ring electrodes (4 mm inside diameter), mounted 10 mm apart on a perspex rod. The bottom ligature was passed through a fine hole in a nylon screw at the base of the rod. By turning the screw the muscle strip could be drawn exactly through the electrode rings. The electrode assemblies were then placed in the jacketed organ baths as described above and the upper muscle ligatures attached to the transducer to measure tension. Both the vas deferens and the bladder smooth muscles responded to the field stimulation with consistent repeatable contractions. It is believed that these contractions are calcium-dependent via the operation of slow calcium channels. The effect of ABA on these contractions was tested by adding a concentrated solution thereof to the perfusion solution to give an overall ABA concentration of $10^{-5}$M. In the two vas deferens studies the slow calcium channel blocker nifedipine was then added in the same manner to produce a concentration of $10^{-7}$M. The results are shown in Figure 1. A = rat urinary bladder detrusor muscle strips; B = rat vas deferens smooth muscle; and C = rat vas deferens smooth muscle.

As can be seen, ABA, at a concentration$10^{-5}$M enhanced field stimulation responses in both muscles by between 25 and 40% and this activity was inhibited by the slow calcium channel blocker nifedipine.

## Example 2

(A) Rat urinary bladder detrusor muscle strips were tested in a high-K saline prepared by substitution of sodium compounds in the Krebs' solution by equimolar amounts of potassium compounds to give a K+ concentration of 60 mM. At this level of depolarization it is believed that the fast calcium channels are voltage-inactivated. As shown in Figure 2A, the exposure to the high-K Krebs solution elicited a contracture response consisting of an initial large phasic component and a prolonged smaller tonic contraction. The effect of ABA on this contraction was tested by (B) adding a concentrated solution thereof to the Krebs' perfusion solution (to give $10^{-6}$ M concentration of ABA) 15 minutes before replacement of the Krebs' solution with the high-K solution; as shown in Figure 2B, 50% enhancement of contracture tension was obtained;

(C) by adding ABA to the high-K saline to a concentration of $10^{-6}$ M just after the initial phasic response; as shown in Figure 2C, there was considerable enhancement of the tonic phase.

Both contracture tension and tonic tension are thought to be dependent on slow calcium channel activity.

Example 3

The contractile force in flounder heart was measured before and after addition of ABA to the perfusion solution. The results are shown in Figure 3. As shown in Figure 3A, ABA at $10^{-6}$ was found to augment contractile force; this action was inhibited by nifedipine. Figure 3B shows the effect on stroke volume (upper trace) and contractile force (lower trace) after 15 minutes exposure to ABA.

Heart contraction is generally believed to be a slow calcium channel activity and its enhancement in Figure 3A gives further support to the idea that ABA is a calcium agonist. The pronounced arrhythmia in Figure 3B is consistent with this theory and is probably caused by excess calcium entry to the heart cells.

Example 4

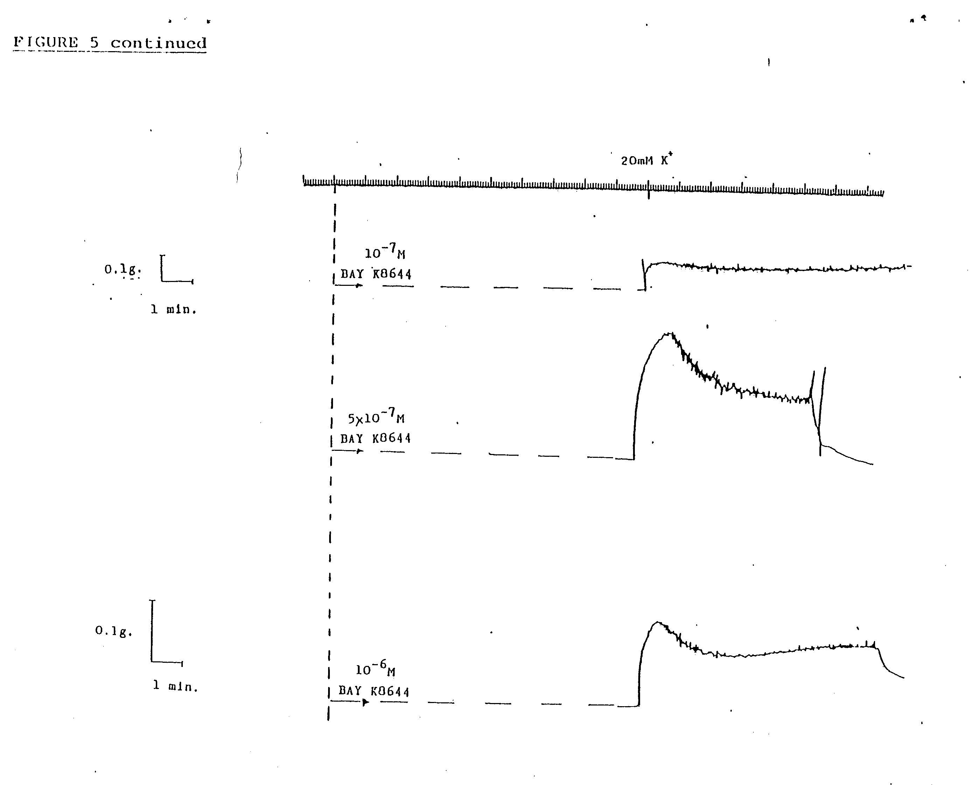

To date the only successful calcium channel agonist is the Bayer compound BAY K 8644. To confirm the view that ABA may have calcium channel agonist activity, the effects of ABA and BAY K 8644 were compared in parallel trials on similar tissues under identical conditions to establish dose-response relationships. These results are shown in Figures 4 and 5. BAY K 8644 and ABA were found to have identical activity in enhancing K+-induced (20 mM K) contracture tension in rat vas deferens smooth muscle (partly depolarized). In Figure 4, the left hand panel shows three control K contractures and the right hand panel shows ABA action on the K contracture. The threshold of ABA action was about 5 x $10^{-9}$ M for 100% enhancement of response. In Figure 5, the left hand panel shows two control contractures and the right hand panel shows the action of BAY K 8644 on contracture tension. The BAY K 8644 threshold for 100% enhancement of K contracture tension was $10^{-9}$ to 5 x $10^{-10}$ M, roughly one order of magnitude greater than ABA. Thus, while BAY K 8644 appeared in these tests to be slightly more potent, ABA seemed clearly to exert a similar action.

Even more recent findings show that ABA enhances potassium-stimulated microsomal calcium efflux and enhances field stimulation response. What these results suggest is that ABA has enhanced cellular calcium levels to produce these enhanced responses of visceral and cardiac muscles.

## Claims

1. A pharmaceutical composition for changing calcium flux across cellular membranes in man or animals comprising a pharmaceutically suitable natural or synthetic plant growth regulating compound having calcium-regulatory activity in the human or animal body or a derivative or metabolite thereof having calcium-regulatory activity in the human or animal body, with the exception of ABA, and a pharmaceutically acceptable carrier.

2. A composition as claimed in Claim 1 wherein the compound has calcium agonist activity.

3. A composition as claimed in Claim 1 wherein the compound has calcium antagonist activity.

4. A composition as claimed in Claim 1 wherein the compound is selected from those having the formulae:

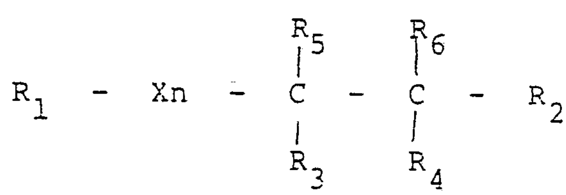

or

$R_1$ -Y - $R_2$

wherein $R_1$ is an alkyl group of up to seven carbons or a saturated or unsaturated six membered carbon ring, which may be substituted; $R_2$ is a cyano group, a thio-or hydroxy-lower alkyl group, or a group containing a carbonyl; $R_3$ is hydrogen, lower alkyl, or trifluoromethyl; $R_4$ is hydrogen, lower alkyl or halide; $R_5$ is hydrogen or hydroxy; $R_6$ is hydrogen, carboxy or carboxy lower alkyl, or $R_5$ and $R_6$ together form a bond between the carbons to which they are attached or with the carbons to which they are attached form a carbocyclic group; or $R_2$ and $R_6$ together with the carbon to which they are bonded form a heterocyclic ring; n is 0 or 1, with the proviso that when n is 0, $R_2$ is an

acyl group; X is $-CH_2-$, $-CH=CH-$, $-CH_2-CH_2-$, $-CH_2-NH-$, $-CH_2-O-$, $-CH_2-S-$or -NH-CO-; and Y is a carbocyclic group bonded to $R_1$ and $R_2$ through a single carbon.

5. A composition as claimed in Claim 4 wherein the compound has the following substituents

$R_1$ is selected from the group consisting of (i) saturated and unsaturated six-membered carbocyclic groups, which may be substituted or unsubstituted, and (ii) lower alkyl groups of up to seven carbons;

n is an integer selected from 0 and 1;

X is selected from the group consisting of $-CH_2-$, $-CH=CH-$, $-CH_2-NH-$, $-CH_2-O-$, $-CH_2-S-$, $-CH_2-CH_2-$, -NH-CO-;

$R_2$ is selected from the group consisting of (i) carboxyl and lower alkyl or phenyl esters and salts thereof, (ii) cyano, (iii) carbamoyl groups of the formula $CON(R_7R_8)$ wherein $R_7$ and $R_8$ are independently lower alkyl or hydrogen, or with the nitrogen atom to which they are attached, $R_7$ and $R_8$ form a saturated or unsaturated heterocyclic ring, (iv) thio-or hydroxy-lower alkyl, and (v) when n is 0, an acyl group of the formula $COR_9$ wherein $R_9$ is lower alkyl;

$R_3$ is hydrogen, trifluoromethyl or lower alkyl;

$R_4$ is selected from the group consisting of hydrogen, lower alkyl and halide;

$R_5$ is hydrogen or hydroxy;

$R_6$ is hydrogen, carboxy, or carboxy lower alkyl;

or $R_5$ and $R_6$ form a bond between the carbons to which they are bonded or together with the carbons to which they are bonded form a saturated or unsaturated carbocyclic group of three or six members;

or $R_2$ and $R_6$ together with the carbon to which they are bonded form a heterocyclic group of up to six members, having therein at least one carbonyl group and nitrogen as the heteroatom;

Y is a three to six membered carbocyclic group bonded to $R_1$ and $R_2$ through a single carbon of the ring.

6. A composition as claimed in Claim 1 wherein the compound is a derivative of ABA.

7. A pharmaceutically suitable natural or synthetic plant growth regulating compound, derivative or metabolite thereof having calcium-regulatory activity in the human or animal body, with the exception of ABA, for use in the therapeutic or prophylactic treatment of, or for relief of symptoms of, a disorder of the human or animal body susceptible to treatment by increase or decrease of the calcium flux across a cellular membrane or for use in surgery where such increase or decrease is required.

8. A compound as claimed in Claim 7 which has calcium agonist effect.

9. A compound as claimed in Claim 7 which has calcium antagonist effect.

10. A compound as claimed in Claim 7 wherein the cellular membrane across which flux is changed is the smooth or cardiac muscle membrane.

11. A compound as claimed in Claim 7 for use in therapeutic or prophylactic treatment of loss of bladder tone, left ventricular failure, swelling of the mucous membrane, disease involving depression of blood sugar or incorrect salt or fluid balance, a hypotensive circulatory condition or liver dysfunction, or in the temporary restriction of peripheral blood flow during a surgical procedure.

12. A compound as claimed in Claim 7 for use in therapeutic or prophylactic treatment of hypertension, angina, cardiovascular disease or cardiovascular system disturbances, for the correction of spasm in the major blood vessels or correction of cardiac arrythmia or as a peripheral vasodilator.

13. A compound as claimed in any one of claims 7 to 12, which has the formula:

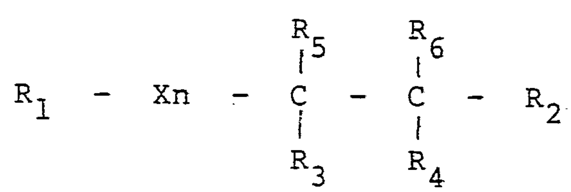

$R_1$ -Y - $R_2$

wherein $R_1$ is an alkyl group of up to seven carbons or a saturated or unsaturated six membered carbon ring, which may be substituted; $R_2$ is a cyano group, a thio-or hydroxy-lower alkyl group, or a group containing a carbonyl, most commonly a carboxyl group; $R_3$ is hydrogen, trifluoromethyl or lower alkyl; $R_4$ is hydrogen, lower alkyl or halide; $R_5$ is hydrogen or hydroxy; $R_6$ is hydrogen, carboxy or carboxy lower alkyl, or $R_5$ and $R_6$ together form a bond between the carbons to which they are attached or with the carbons to which they are attached form a carbon ring; or $R_2$ and $R_6$ together with the carbon to which they are bonded form a heterocyclic ring; n is 0 or 1, with the proviso that when n is 0, $R_2$ is an acyl group; X is $-CH_2-$, $-CH=CH-$, $-CH_2CH_2-$, $-CH_2-NH-$, $-CH_2-O-$, $-CH_2-S-$or -NH-CO-; and Y is a cyclic group bonded to $R_1$ and $R_2$ through a single carbon.

14. The use of a pharmaceutically suitable natural or synthetic plant growth regulating compound, derivative or metabolite thereof having calcium-regulatory activity in the human or animal body for the manufacture of a medicament for the therapeutic or prophylactic treatment of, or for relief of symptoms of, a disorder of the human or animal body susceptible to treatment by increase or decrease of the calcium flux across a cellular membrane or for use in surgery where such increase or decrease is required.

15. The use as claimed in Claim 14 wherein the compound has the formula

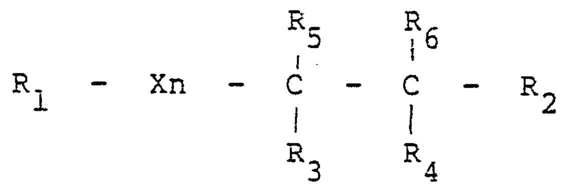

or

$R_1$ -Y - $R_2$

wherein $R_1$ is an alkyl group or up to seven carbons or a saturated or unsaturated six membered carbon ring, which may be substituted; $R_2$ is a cyano group, a thio-or hydroxy-lower alkyl group, or a group containing a carbonyl, most commonly a carboxyl group; $R_3$ is hydrogen, trifluoromethyl or lower alkyl; $R_4$ is hydrogen, lower alkyl or halide; $R_5$ is hydrogen or hydroxy; $R_6$ is hydrogen, carboxy or carboxy lower alkyl, or $R_5$ and $R_6$ together form a bond between the carbons to which they are attached or with the carbons to which they are attached form a carbocyclic group; or $R_2$ and $R_6$ together with the carbon to which they are bonded form a heterocyclic ring; n is 0 or 1, with the proviso that when n is 0, $R_2$ is an acyl group; X is $-CH_2-$, $-CH=CH-$, $-CH_2CH_2-$, $-CH_2-NH-$, $-CH_2-O-$, $-CH_2-S-$or -NH-CO-; and Y is a carbocyclic group bonded to $R_1$ and $R_2$ through a single carbon.

16. The use as claimed in Claim 15, wherein the compound has the following substituents:

$R_1$ is selected from the group consisting of (i) saturated and unsaturated six-membered carbocyclic groups, which may be substituted or unsubstituted, and (ii) lower alkyl groups of up to seven carbons;

n is an integer selected from 0 and 1;

X is selected from the group consisting of $-CH_2-$, $-CH=CH-$, $-CH_2-NH-$, $-CH_2-O-$, $-CH_2-S-$, $-CH_2-CH_2-$, -NH-CO-;

$R_2$ is selected from the group consisting of (i) carboxyl and lower alkyl or phenyl esters and salts thereof, (ii) cyano, (iii) carbamoyl groups of the formula $CON(R_7R_8)$ wherein $R_7$ and $R_8$ are independently lower alkyl or hydrogen, or with the nitrogen atom to which they are attached, $R_7$ and $R_8$ form a saturated or unsaturated heterocyclic ring, (iv) thio-or hydroxy-lower alkyl, and (v) when n is 0, an acyl group of the formula $COR_9$ wherein $R_9$ is lower alkyl;

$R_3$ is hydrogen, trifluoromethyl or lower alkyl;

$R_4$ is selected from the group consisting of hydrogen, lower alkyl and halide;

$R_5$ is hydrogen or hydroxy;

$R_6$ is hydrogen, carboxy, or carboxy lower alkyl;

or $R_5$ and $R_6$ form a bond between the carbons to which they are bonded or together with the carbons to which they are bonded form a saturated or unsaturated carbocyclic group of three or six members;

or $R_2$ and $R_6$ together with the carbon to which they are bonded form a heterocyclic group of up to six members, having therein at least one carbonyl group and nitrogen as the heteroatom;

Y is a three to six membered carbocyclic group bonded to $R_1$ and $R_2$ through a single carbon of the ring.

17. A pharmaceutical composition for changing calcium flux across cellular membranes in man or animals comprising a pharmaceutically suitable natural or synthetic plant growth regulating compound having calcium-regulatory activity in the human or animal body or a derivative or metabolite thereof having calcium-regulatory activity in the human or animal body, and a pharmaceutically acceptable carrier, the composition being in dosage unit form and containing up to 250 mg of active ingredient per dosage unit.

18. A pharmaceutical composition as claimed in Claim 17, which contains up to 60 mg of active ingredient per dosage unit.

Claims for the following contracting states: Austria, Greece and Spain

1. A process for the preparation of a pharmaceutical composition for changing calcium flux across cellular membranes in man or animals, which comprises bringing a pharmaceutically suitable natural or synthetic plant growth regulating compound having calcium-regulatory activity in the human or animal body

or a derivative or metabolite thereof having calcium-regulatory activity in the human or animal body, with the exception of ABA, together with a pharmaceutically acceptable carrier, into the form of a pharmaceutical composition.

2. A process as claimed in Claim 1 wherein the compound has calcium agonist activity.

3. A process as claimed in Claim 1 wherein the compound has calcium antagonist activity.

4. A process as claimed in Claim 1 wherein the compound is selected from those having the formulae:

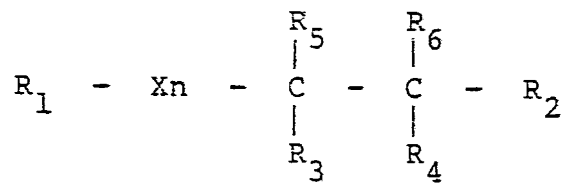

or

$R_1$ -Y - $R_2$

wherein $R_1$ is an alkyl group of up to seven carbons or a saturated or unsaturated six membered carbon ring, which may be substituted; $R_2$ is a cyano group, a thio-or hydroxy-lower alkyl group, or a group containing a carbonyl; $R_3$ is hydrogen, lower alkyl, or trifluoromethyl; $R_4$ is hydrogen, lower alkyl or halide; $R_5$ is hydrogen or hydroxy; $R_6$ is hydrogen, carboxy or carboxy lower alkyl, or $R_5$ and $R_6$ together form a bond between the carbons to which they are attached or with the carbons to which they are attached form a carbocyclic group; or $R_2$ and $R_6$ together with the carbon to which they are bonded form a heterocyclic ring; n is 0 or 1, with the proviso that when n is 0, $R_2$ is an acyl group; X is $-CH_2-$, $-CH=CH-$, $-CH_2-CH_2-$, $-CH_2-NH-$, $-CH_2-O-$, $-CH_2-S-$or $-NH-CO-$; and Y is a carbocyclic group bonded to $R_1$ and $R_2$ through a single carbon.

5. A process as claimed in Claim 4 wherein the compound has the following substituents

$R_1$ is selected from the group consisting of (i) saturated and unsaturated six-membered carbocyclic groups, which may be substituted or unsubstituted, and (ii) lower alkyl groups of up to seven carbons;

n is an integer selected from 0 and 1; X is selected from the group consisting of $-CH_2-$, $-CH=CH-$, $-CH_2-NH-$, $-CH_2-O-$, $-CH_2-S-$, $-CH_2-CH_2-$, $-NH-CO-$;

$R_2$ is selected from the group consisting of (i) carboxyl and lower alkyl or phenyl esters and salts thereof, (ii) cyano, (iii) carbamoyl groups of the formula $CON(R_7R_8)$ wherein $R_7$ and $R_8$ are independently lower alkyl or hydrogen, or with the nitrogen atom to which they are attached, $R_7$ and $R_8$ form a saturated or unsaturated heterocyclic ring, (iv) thio-or hydroxy-lower alkyl, and (v) when n is 0, an acyl group of the formula $COR_9$ wherein $R_9$ is lower alkyl;

$R_3$ is hydrogen, trifluoromethyl or lower alkyl;

$R_4$ is selected from the group consisting of hydrogen, lower alkyl and halide;

$R_5$ is hydrogen or hydroxy;

$R_6$ is hydrogen, carboxy, or carboxy lower alkyl;

or $R_5$ and $R_6$ form a bond between the carbons to which they are bonded or together with the carbons to which they are bonded form a saturated or unsaturated carbocyclic group of three or six members;

or $R_2$ and $R_6$ together with the carbon to which they are bonded form a heterocyclic group of up to six members, having therein at least one carbonyl group and nitrogen as the heteroatom;

Y is a three to six membered carbocyclic group bonded to $R_1$ and $R_2$ through a single carbon of the ring.

6. A process as claimed in Claim 1 wherein the compound is a derivative of ABA.

7. The use of a pharmaceutically suitable natural or synthetic plant growth regulating compound, derivative or metabolite thereof having calcium-regulatory activity in the human or animal body for the manufacture of a medicament for the therapeutic or prophylactic treatment of, or for relief of symptoms of, a disorder of the human or animal body susceptible to treatment by increase or decrease of the calcium flux across a cellular membrane or for use in surgery where such increase or decrease is required.

8. The use as claimed in Claim 7 wherein the compound has the formula

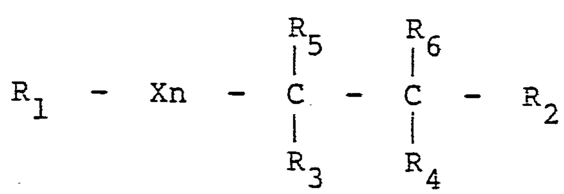

or

$R_1$ -Y - $R_2$

wherein $R_1$ is an alkyl group of up to seven carbons or a saturated or unsaturated six membered carbon ring, which may be substituted; $R_2$ is a cyano group, a thio-or hydroxy-lower alkyl group, or a group containing a carbonyl, most commonly a carboxyl group; $R_3$ is hydrogen, trifluoromethyl or lower alkyl; $R_4$ is hydrogen, lower alkyl or halide; $R_5$ is hydrogen or hydroxy; $R_6$ is hydrogen, carboxy or carboxy lower alkyl, or $R_5$ and $R_6$ together form a bond between the carbons to which they are attached or with the carbons to which they are attached form a carbocyclic group; or $R_2$ and $R_6$ together with the carbon to which they are bonded form a heterocyclic ring; n is 0 or 1, with the proviso that when n is 0, $R_2$ is an acyl group; X is $-CH_2-$, $-CH=CH-$, $-CH_2CH_2-$, $-CH_2-NH-$, $-CH_2-O-$, $-CH_2-S-$or -NH-CO-; and Y is a carbocyclic group bonded to $R_1$ and $R_2$ through a single carbon.

9. The use as claimed in Claim 8, wherein the compound has the following substituents:

$R_1$ is selected from the group consisting of (i) saturated and unsaturated six-membered carbocyclic groups, which may be substituted or unsubstituted, and (ii) lower alkyl groups of up to seven carbons;

n is an integer selected from 0 and 1;

X is selected from the group consisting of $-CH_2-$, $-CH=CH-$, $-CH_2-NH-$, $-CH_2-O-$, $-CH_2-S-$, $-CH_2-CH_2-$, -NH-CO-;

$R_2$ is selected from the group consisting of (i) carboxyl and lower alkyl or phenyl esters and salts thereof, (ii) cyano, (iii) carbamoyl groups of the formula $CON(R_7R_8)$ wherein $R_7$ and $R_8$ are independently lower alkyl or hydrogen, or with the nitrogen atom to which they are attached, $R_7$ and $R_8$ form a saturated or unsaturated heterocyclic ring, (iv) thio-or hydroxy-lower alkyl, and (v) when n is 0, an acyl group of the formula $COR_9$ wherein $R_9$ is lower alkyl;

$R_3$ is hydrogen, trifluoromethyl or lower alkyl;

$R_4$ is selected from the group consisting of hydrogen, lower alkyl and halide;

$R_5$ is hydrogen or hydroxy;

$R_6$ is hydrogen, carboxy, or carboxy lower alkyl;

or $R_5$ and $R_6$ form a bond between the carbons to which they are bonded or together with the carbons to which they are bonded form a saturated or unsaturated carbocyclic group of three or six members;

or $R_2$ and $R_6$ together with the carbon to which they are bonded form a heterocyclic group of up to six members, having therein at least one carbonyl group and nitrogen as the heteroatom;

Y is a three to six membered carbocyclic group bonded to $R_1$ and $R_2$ through a single carbon of the ring.

10. A process for the preparation of a pharmaceutical composition for changing calcium flux across cellular membranes in man or animals, which comprises bringing a pharmaceutically suitable natural or synthetic plant growth regulating compound having calcium-regulatory activity in the human or animal body or a derivative or metabolite thereof having calcium-regulatory activity in the human or animal body, together with a pharmaceutically acceptable carrier, into the form of a pharmaceutical composition in dosage unit form containing up to 250 mg of active ingredient per dosage unit.

11. A process as claimed in Claim 10, wherein the composition contains up to 60 mg of active ingredient per dosage unit.

FIGURE 1

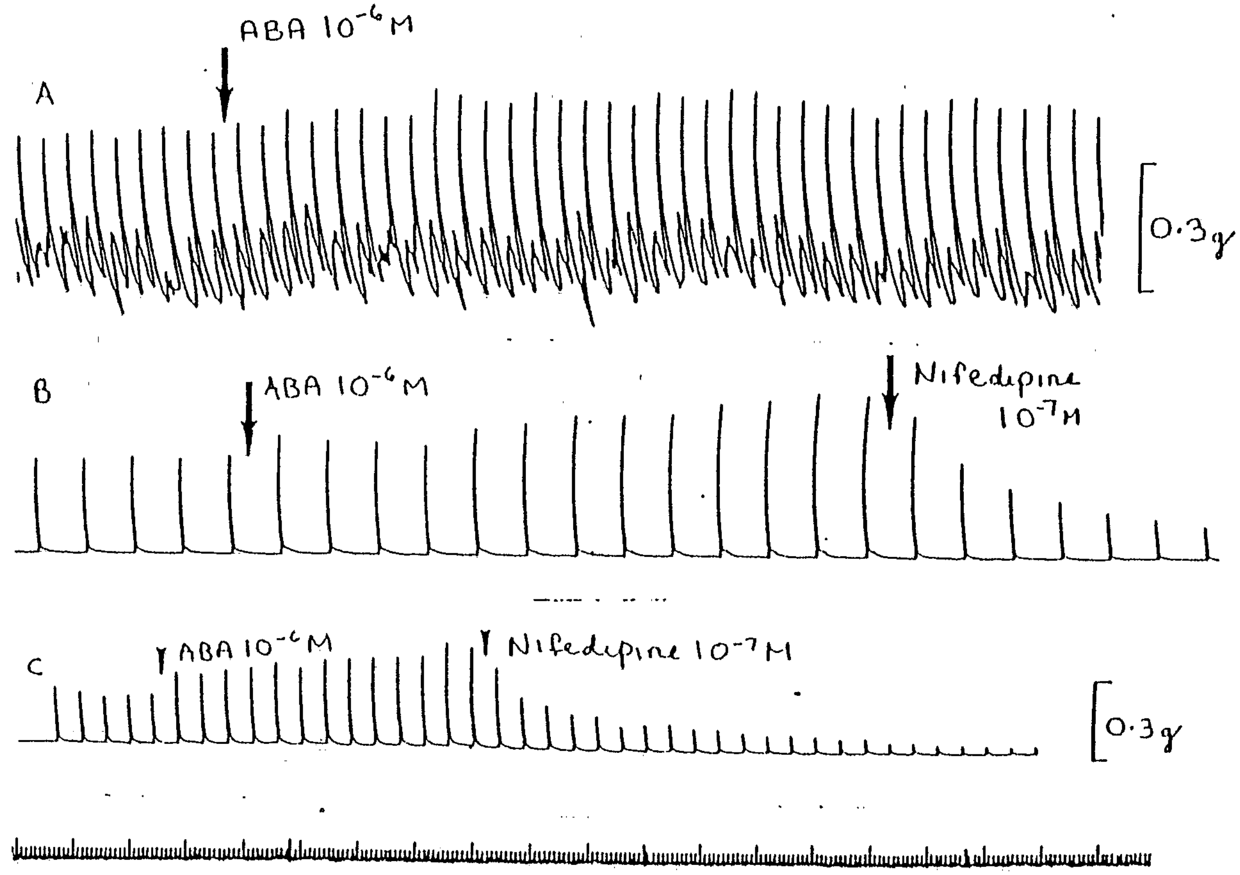
ABA 10⁻⁶M
A
0.3g
B
ABA 10⁻⁶M
Nifedipine 10⁻⁷M
C
ABA 10⁻⁶M
Nifedipine 10⁻⁷M
0.3g

FIGURE 2

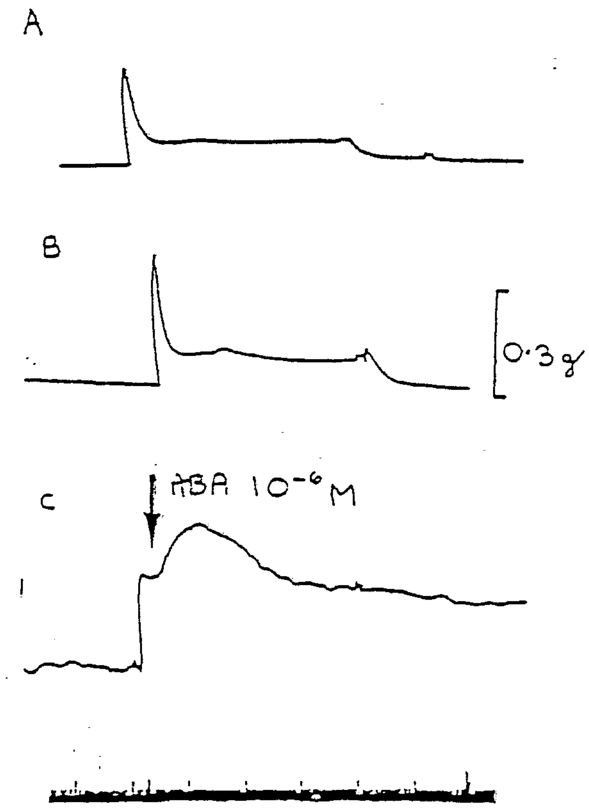

FIGURE 3

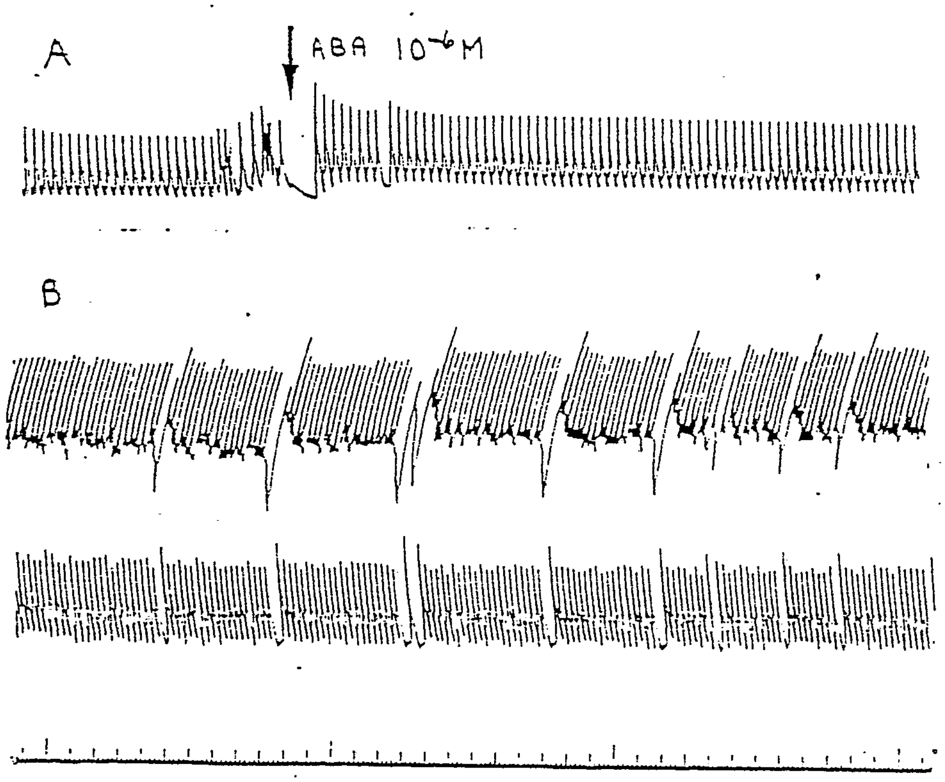

FIGURE 4

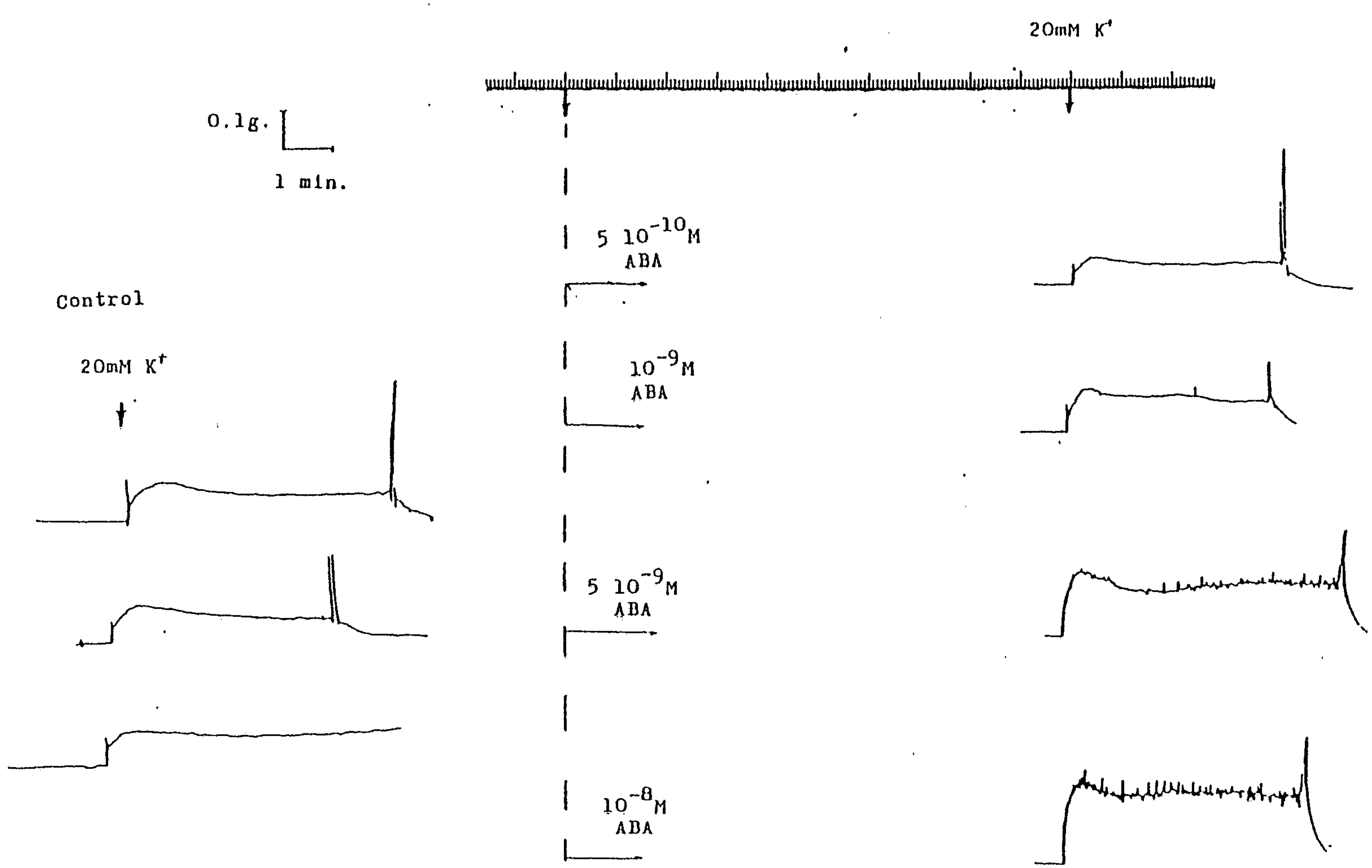

0.1g.
1 min.
Control
20mM K+
20mM K+
5 10-10M ABA
10-9M ABA
5 10-9M ABA
10-8M ABA

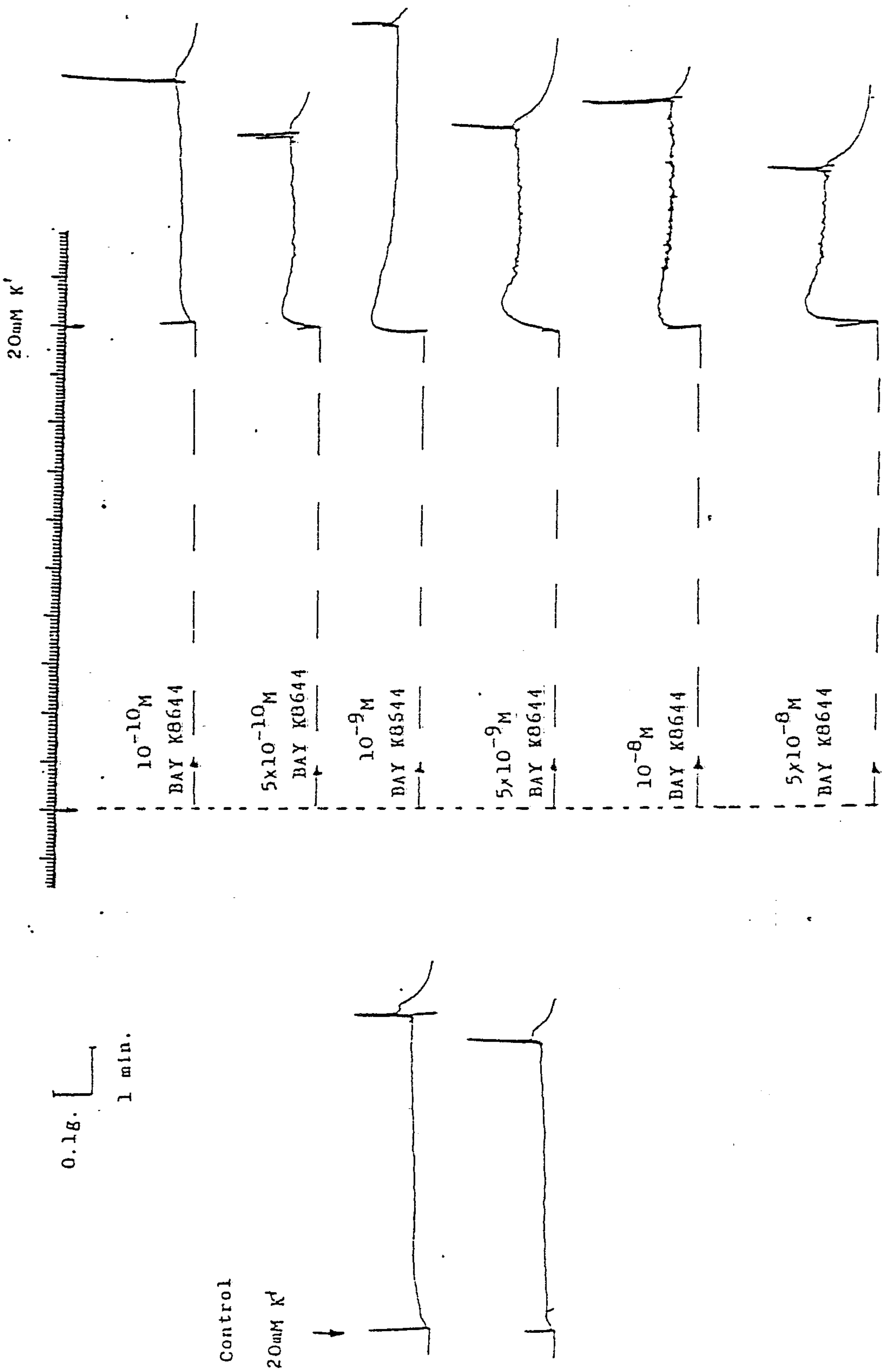
20mM K+
10^-10 M BAY K8644
5x10^-10 M BAY K8644
10^-9 M BAY K8644
5x10^-9 M BAY K8644
10^-8 M BAY K8644
5x10^-8 M BAY K8644
0.1g.
1 min.
Control
20mM K+

FIGURE 5

FIGURE 5 continued